# EUROPEAN PATENT APPLICATION

(11) **EP 1 166 736 A2**
(43) Date of publication of application: **02.01.2002**
(21) Application number: 01305011.7
(22) Date of filing: 08.06.2001
(51) Int. Cl.: A61F 13/56, A61F 13/62, A61F 13/49

(54) **Disposable diaper**

(30) Priority: 19.06.2000 JP 2000183845
(71) Applicant: UNI-CHARM CORPORATION, Kawanoe-shi Ehime-ken (JP)
(72) Inventor: Shingu, Yoshikazu, Uni-Charm Corp. Tech. center, Mitoyo-gun, Kagawa-ken, 769-1602 (JP); Mukai, Hirotomo, Uni-Charm Corp.Technical center, Mitoyo-gun, Kagawa-ken, 769-1602 (JP)
(74) Representative: Parry, Christopher Stephen

(57) **Abstract**

A disposable diaper 1 has a pair of wings 12, and the wings 12 are formed with a nonwoven fabric and the nonwoven fabric partially extends outward in a circumferential direction to form fastener sections 21 which are, in turn, provided on its inner surfaces with male members 22 of mechanical fasteners. The nonwoven fabric is formed on the inner surface thereof with a plurality of fine fusion spots 20 so that the number of the fine fusion spots 20 per unit area of the inner surfaces is larger in the fastener sections 21 and first regions 41 than in second regions 42 extending inside the first regions 41.

## Description

This invention relates to a disposable diaper.

Conventional disposable diapers are provided in its rear waist region with a pair of wings formed with a nonwoven fabric. In those diapers, the wings are formed with fastener sections serving to connect the front and rear waist regions to each other. The fastener sections are provided on inner surfaces thereof with adhesive zones or male members of so-called mechanical fasteners so that these adhesive zones or male members may be detachably fixed to the front waist region.

The fastener sections may be formed, for example, by fixing the male members of the mechanical fasteners to the nonwoven fabric of the wings. The diaper can be put on a wearer's body by pressing the male members against target zone or zones formed on the outer surface of the front waist region and thereby connecting the front and rear waist regions to each other. However, considerably high pressure may be required to anchor the male members on the target zone or zones, depending on particular characteristics of the male members and target zone or zones. The male members are generally of high stiffness and therefore the male members each having an excessively large size would undesirably stimulate the wearer's skin and, in addition, make it difficult to peel these male members off from the target zone or zones. While relatively small-sized male members are sometimes used to overcome such problems, it is not easy to grasp these small male members and then to press them against the target zone or zones in quick and effective manner.

In view of the problems as have been described above, it is an object of this invention to improve the known disposable diapers so that the male members may be easily anchored on the target zone or zones even if the fastener sections comprise the nonwoven fabric and the male members of the mechanical fasteners fixed to the nonwoven fabric.

According to this invention, there is a disposable diaper comprising a liquid-pervious topsheet, a liquid-impervious backsheet and a liquid-absorbent core disposed between these two sheets so as to configure a front waist region, a rear waist region and a crotch region extending between these two waist regions in a longitudinal direction of the diaper wherein the rear waist region is formed on transversely opposite side portions thereof with wings extending outward in a circumferential direction intersecting the longitudinal direction and the wings are formed with fastener sections extending outward in the circumferential direction and provided on inner surfaces thereof with male members as components of so-called mechanical fasteners.

The improvement according to this invention is in that the wings are formed with a nonwoven fabric made of thermoplastic synthetic fibers, the nonwoven fabric partially extending outward from circumferentially outer side regions of the wings to form the fastener sections which are, in turn, provided on inner surfaces thereof with the male members of the mechanical fasteners, and the wings are formed on the inner surfaces thereof with a plurality of fine fusion spots at which the fibers are fused together so that the number of the fine fusion spots per unit area of the wings nonwoven fabric is larger in the outer side regions of the wings than inner regions of the wings extending inward from the outer side regions.
Fig. 1 is a plan view showing an inner side of the disposable diaper as partially broken away;
Fig. 2 is a plan view showing an outer side of the disposable diaper;
Fig. 3 is a fragmentary diagram showing a rear wing in an enlarged scale; and
Fig. 4 is a sectional view taken along a line IV - IV in Fig. 3.

Details of the disposable diaper according to this invention will be more fully understood from the description given hereunder with reference to the accompanying drawings.

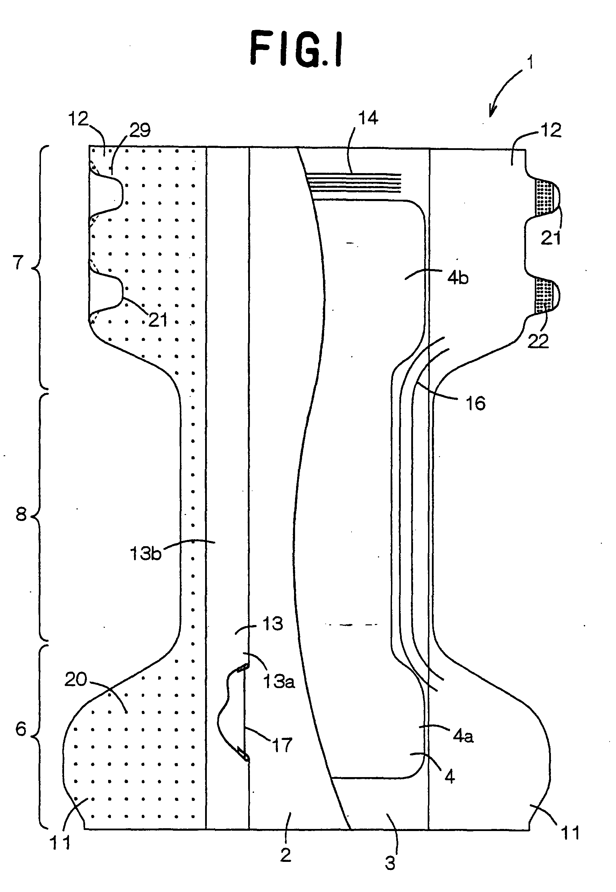

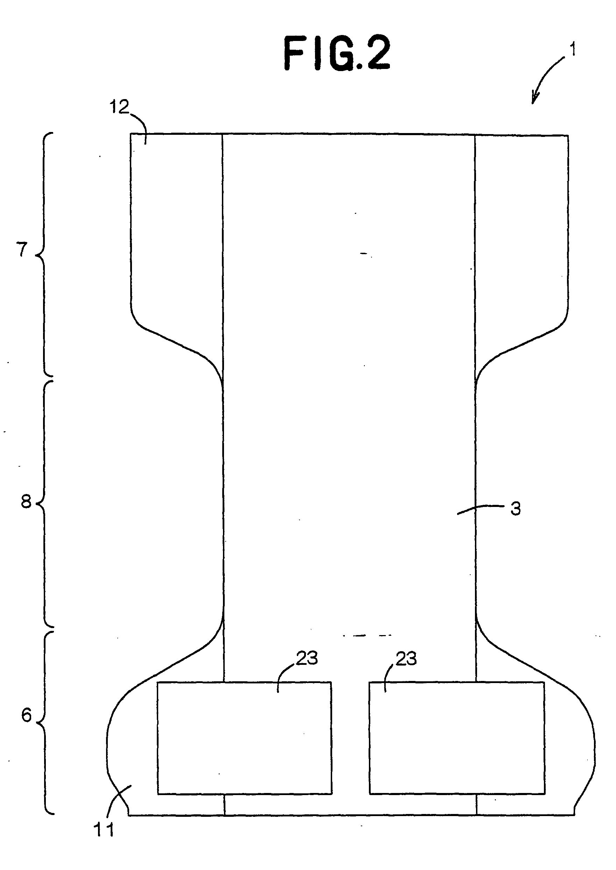

Fig. 1 is a plan view showing the inner side of diaper 1 as partially broken away and Fig. 2 is a plan view showing the outer side of this diaper 1. The diaper 1 has a liquid-pervious topsheet 2, a liquid-impervious backsheet 3 and a liquid-absorbent core 4 disposed between these two sheets 2, 3. The diaper 1 is longitudinally (i.e., vertically as viewed in these figures) composed of a front waist region 6, a rear waist region 7 and a crotch region 8 extending between these two waist regions 6, 7. The front and rear waist regions 6, 7 are respectively formed with front wings 11 and rear wings 12 extending circumferentially outward from transversely opposite side edges thereof. In the front and rear wings 11, 12 and transversely opposite side portions of the crotch region 8 are formed with a plurality of depressed fine fusion spots 20. Along an outer end of the rear waist region 7, a plurality of elastic members 14 extend circumferentially of the region 7 to be associated with a waist- opening. The crotch region 8 is provided along transversely opposite side edges thereof with a plurality of elastic members 16 extending longitudinally of the region 8 to be associated with respective leg-openings. These elastic members 14, 16 are disposed between the top- and backsheets 2, 3 or separately prepared sheets bonded to these top- and backsheets 2, 3, respectively, to extend and bonded under tension to one of these sheets.

A pair of ribbon-like barrier cuffs 13 longitudinally extend on the inner surface of the diaper 1 in parallel to transversely opposite side edges of the diaper 1, respectively. Each of the barrier cuffs 13 has its outer side edge portion 13b as well as its longitudinally opposite end portions fixed to the inner surface of the diaper 1 and its inner side edge portion 13a not fixed to the inner surface of the diaper 1. More specifically, the inner side edge portion 13a is folded back in envelope-like manner to wrap a longitudinally extending elastic member 17 biasing the cuff 13 to rise on the inner surface of the diaper 1. The elastic member 17 is bonded under tension to the inner surface of the barrier cuff 13 at least at the longitudinally opposite end portions so that contraction of the elastic member 17 may cause the barrier cuff 13 to rise on the inner surface of the diaper 1 as the diaper 1 is put on a wearer' s body. Thus, such barrier cuffs 13 are adapted to form a pair of pockets (not shown) opening inwardly of the diaper 1 and thereby to prevent body fluids from leaking.

The transversely opposite side edges of the rear wing 12 partially extend circumferentially outward to form respective pairs of fastener sections 21 spaced apart from each other vertically as viewed in Figs. 1 and 2. Each of the fastener sections 21 is provided on its inner surface with a male member 22 as a component of a mechanical fastener so as to extend longitudinally thereof. Fig. 1 shows the left side fastener sections 21 as folded onto the inner surface of the rear wing 12 and the right side fastener sections 21 as extending circumferentially outward. As will be seen in Fig. 2, all the fastener sections 21 are folded onto the inner surfaces of the wing 12. These fastener sections 21 can be peelably anchored on the associated female members 23 at appropriate positions thereof to connect the front and rear waist regions 6, 7 to each other.

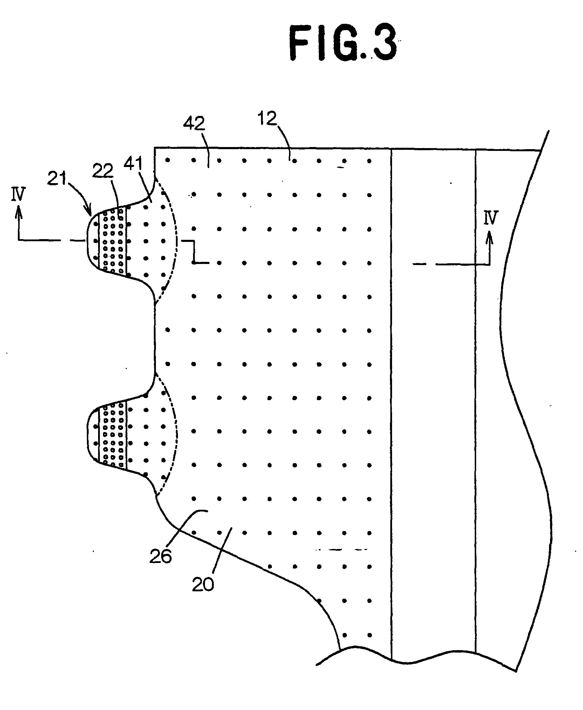

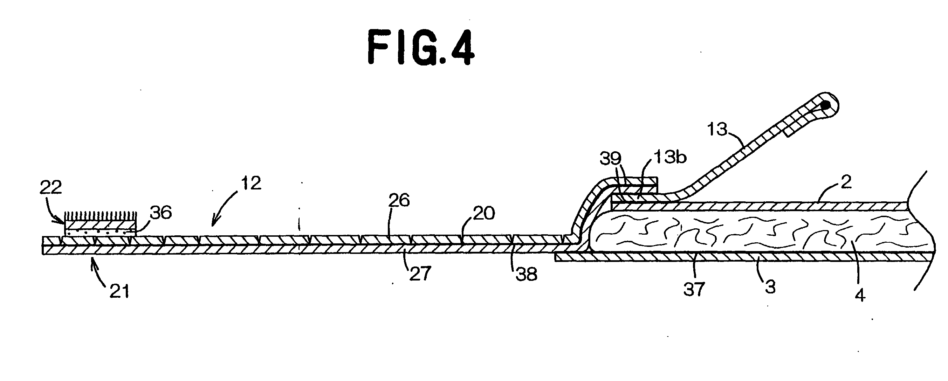

Fig. 3 is a fragmentary plan view of the diaper 1 showing the rear wing 12 in an enlarged scale and Fig. 4 is a sectional view taken along a line IV - IV in Fig. 3. Referring to Figs. 3 and 4, the fastener sections 21 extend outward in a circumferential direction. The rear wing 12 comprises an inside nonwoven fabric layer 26 defining the inner side of the diaper 1 and an outer side nonwoven fabric layer 27 defining the outer side of the diaper 1. These two nonwoven fabric layers 26, 27 are bonded to each other by means of hot melt adhesive 38. The fastener sections 21 extending from the rear wing 12 are formed as partial extensions of the inner and outer side nonwoven fabric layers 26, 27 and the male members 22 bonded to the inner side nonwoven fabric layer 26 by means of adhesive 36. These male members 22 are peelably engaged with respective fastener holding zones (out side regions) 41 as the fastener sections 21 are folded back onto the inner surface of the diaper 1 (See Fig. 1).

Each of the rear wings 12 is formed on outer side region 41 indicated by chain lines inclusive of the fastener sections 21 and the vicinity thereof and inner side region 42 extending inward from the outer side region 41 with a plurality of fine fusion spots 20. These fine fusion spots 20 may be formed by locally heating the inner side nonwoven fabric layer 26, more preferably both the inner side nonwoven fabric 26 and the outer side nonwoven fabric layer 27 under pressure so that the component fibers of the inner side nonwoven fabric layer 26 may be fused together or the component fibers of the inner side nonwoven fabric layer 26 and the outer side nonwoven fabric layer 27 may be fused together. The fusion spots 20 function to improve the tensile strength as well as the stiffness but to deteriorate the breathability of the inner and outer side nonwoven fabric layers 26, 27. Taking account of this, an occupancy ratio of the fusion spots 20 per unit area of the rear wings 12 may be adjusted to be relatively high in the outer side regions 41 and relatively low in the inner side regions 42, more preferably to be 10 ∼ 70 % in the outer side regions 41 and to be at least 20 % lower than the occupancy ratio in the outer side regions 41, i.e., 0 ∼ 30 %. In the case of the inner and outer side nonwoven fabric layers 26, 27 made of polypropylene fiber having a basis weight of 10 ∼ 100 g/m², for example, an occupancy of the fusion spots 20 each having a diameter 0.1 ∼ 1.5 mm to the total area of the rear wings 12 may be set to about 30 % in the outer region 41 and about 10 % in the inner region 42.

The outer side regions 41 arranged as has been described above are more stiff than the inner side regions 42 continuous to the outer side regions 41 and larger than the male members 22. When it is desired to anchor the male members 22 on the female members 23 provided on the front waist region 6, the outer side regions 41 can be easily grasped even if the male members 22 are of relatively small size and the outer side regions 41 may be simply pressed against the female members 23 to anchor the male members 22 on the respective female members 23. On the other hand, in the inner side regions 42 in which the occupancy ratio of the fusion spots 20 is relatively low, the inner and outer side nonwoven fabric layers 26, 27 can maintain flexibility and breathability peculiar to a nonwoven fabric and the region of the diaper 1 surrounding the wearer' s trunk offers a comfortable touch and is free from stuffiness.

In the diaper 1, the front wings 11 as well as the transversely opposite side portions of the crotch region 8 are constructed in a substantially same manner as the rear wings 12. More specifically, the inner side nonwoven fabric layer 26 and the outer side nonwoven fabric layer 27 are bonded to each other by means of adhesive 38 and, in addition, fused together at the fusion spots 20. In the crotch region 8, the inner side nonwoven fabric layer 26 stiffened due to formation of the fusion spots 20 may sometimes stimulate skin of the diaper wearer. If there is such apprehension, a density at which the fusion spots 20 are distributed in the crotch region 8 may be appropriately lowered. The inner and outer side nonwoven fabric layers 26, 27 forming these front and rear wings 11, 12 and the transversely opposite side portions of the crotch region 8 are bonded to the outer side edge portions 13b of the barrier cuffs 13 preferably formed with liquid-impervious sheets using hot melt adhesive 39 or a heat-sealing technique. The outer side edge portions 13b of the barrier cuffs 13 are bonded to the topsheet 2 using hot melt adhesive 39 or a heat-sealing technique. Adhesive 36 ∼ 39 used to assemble the diaper 1 may be intermittently applied the respective members in the longitudinal direction and/or in the circumferential direction.

It is possible without departing from the scope of this invention to configure the outer side regions 41 so that these regions 41 may extend substantially the same as the fastener section 21. However, the outer side regions 41 extending from the fastener sections 21 to parts of the rear wings 12 avoid an anxiety that the fasteners 21 might be easily torn along proximal ends of the respective fastener sections 21 defining boundary lines between the fastener sections 21 and respective rear wings 12. Shape of the individual fusion spots 20 is not limited to circular shape and may be replaced by the other appropriate shape.

In the embodiments of this invention as have been described above, it is possible to form the rear wings 12, the front wings 11 and the side portions of the crotch region 8, which are defined by the inner and outer nonwoven fabric layers 26, 27, by the inner side nonwoven fabric layer 26 alone. It is also possible to replace the rear wings 12 by the front wings 11 and to replace the front wings 11 by the rear wings 12 for implementation of this invention.

In the disposable diaper according to this invention, the fastener sections are formed by attaching the male members of the mechanical fasteners to the nonwoven fabric of the rear wings. However, user can quickly anchor the male members on the female members as the counterparts of the male members even if the male members are of a relatively small size. This is because the fastener sections and the vicinity thereof are formed with the fusion spots distributed at a sufficiently high density to improve the stiffness thereof.

## Claims

1. A disposable diaper comprising a liquid-pervious topsheet, a liquid-impervious backsheet and a liquid-absorbent core disposed between these two sheets so as to configure a front waist region, a rear waist region and a crotch region extending between these two waist regions in a longitudinal direction of the diaper, said rear waist region being formed on transversely opposite side portions thereof with wings extending outward in a circumferential direction intersecting said longitudinal direction and said wings being formed with fastener sections extending outward in said circumferential direction and provided on inner surfaces thereof with male members as components of mechanical fasteners, wherein:
said wings are formed with a nonwoven fabric made of thermoplastic synthetic fibers, said nonwoven fabric partially extending outward from circumferentially outer side regions of said wings to form said fastener sections which are, in turn, provided on inner surfaces thereof with said male members of the mechanical fasteners; and
said wings are formed on the inner surfaces thereof with a plurality of fine fusion spots at which said fibers are fused together so that the number of said fine fusion spots per unit area of said wings nonwoven fabric is larger in said outer side regions of said wings than inner regions of said wings extending inward from said outer side regions.

2. The diaper according to Claim 1, wherein said outer side regions of said nonwoven fabric are more stiff than said inner side regions.

3. The diaper according to Claim 1, wherein said male members are peelably engaged with said inner side regions.
